# EUROPEAN PATENT APPLICATION

(11) **EP 1 719 494 A1**
(43) Date of publication of application: **08.11.2006**
(21) Application number: 05719059.7
(22) Date of filing: 04.02.2005
(51) Int. Cl.: A61K 6/00

(54) **ADHESIVE COMPOSITION FOR ENAMEL**

(30) Priority: 06.02.2004 JP 2004030991
(71) Applicant: SUN MEDICAL CO., LTD., Shiga-ken 524-0044 (JP)
(72) Inventor: ZENG, Weiping, c/o Sun Medical Co., Ltd., Moriyama-shi, Shiga 524-0044 (JP); TASHIRO, Koichi, c/o Sun Medical Co., Ltd., Moriyama-shi, Shiga 524-0044 (JP); TANAKA, Harumi, c/o Sun Medical Co., Ltd., Moriyama-shi, Shiga 524-0044 (JP)
(74) Representative: Albrecht, Thomas
(86) International application number: PCT/JP2005/002101
(87) International publication number: WO 2005/074862

(57) **Abstract**

An adhesive resin cement composition having excellent adhesion and physical properties without the application of a priming material or an adhesive when it is bonded to the enamel and aesthetic properties.

A photocurable dental enamel adhesive composition comprising (A) a radically polymerizable monomer having an acid group and a structure represented by the following formula (1):

CH₂=CR¹-COO-R²-OCO-R³-COOH (1)

(R¹ is H or CH₃, and R² and R³ are each independently a divalent inert group essentially composed of C and H), (B) a monofunctional radically polymerizable monomer having a molecular weight of 220 or less and a boiling point of 60°C/10 mmHg or more, (C) a carboxylic ester group-containing aromatic amine, (D) a photopolymerization initiator and (E) a bifunctional radically polymerizable monomer and optionally (F) a filler.

## Description

### TECHNICAL FIELD

The present invention relates to a photocurable dental enamel adhesive composition. More specifically, it relates to a photocurable enamel adhesive composition for dental treatment which shows excellent adhesion to the dentine, particularly the enamel, without priming or an adhesive aid, can be used for the dentine and also shows excellent adhesion to dental metals, ceramics and dental resins and aesthetic properties.

### BACKGROUND ART

In dental treatment, there are many cases where the enamel is bonded, such as the covering of a discolored tooth and the bonding of a reforming tool. A glass ionomer cement, resin modified glass ionomer cement or resin cement adhesive is generally used to bond a reforming tool.

There is a case where the glass ionomer and resin modified glass ionomer cements do not require the pre-treatment of the enamel before use. However, since they have low bonding strength to the dentine and their curing mechanism is based on an acid-base reaction, their durability in the environment of the mouth is slightly unreliable due to their defects such as water sensitivity and water absorptivity.

To ensure adhesion and durability, resin cement adhesives are often used to bond a reforming tool. However, the resin cement adhesives must require some pre-treatment before use, such as etching and priming, or etching and the application handling of an adhesive, thereby making a clinical treatment complicated.

Consequently, the development of a resin cement adhesive which is bonded to the enamel easily and surely and has practically high durability is desired.

### DISCLOSURE OF THE INVENTION

It is an object of the present invention to provide an adhesive resin cement composition which has solved the above problems of the prior art and has excellent adhesion and mechanical properties without the application handling of a priming material or an adhesive when it is bonded to the enamel and aesthetic properties.

Other objects and advantages of the present invention will become apparent from the following description.

The inventors of the present invention have conducted intensive studies to solve the above problems and have found that the above object can be attained by a photocurable dental enamel adhesive composition comprising:
(A) a radically polymerizable monomer having an acid group and a structure represented by the following formula (1) :

   CH₂=CR¹-COO-R²-OCO-R³-COOH (1)

   (R¹ is H or CH₃, and R² and R³ are each independently a divalent inert group essentially composed of C and H);
(B) a monofunctional radically polymerizable monomer having a molecular weight of 220 or less and a boiling point of 60°C/10 mmHg or more;
(C) a carboxylic ester group-containing aromatic amine;
(D) a photopolymerization initiator; and
(E) a bifunctional radically polymerizable monomer.

The present invention has been accomplished based on this finding.

### BEST MODE FOR CARRYING OUT THE INVENTION

The photocurable dental enamel adhesive composition of the present invention will be described in detail hereinunder.

Surprisingly, the component (A) provides the adhesive composition with adhesion not only to the dentine but also to dental metals and ceramics and further makes possible the coexistence of an acid group-containing monomer and an amine which has been difficult and the stable storage of a resin cement which is an adhesive composition without exerting a bad influence upon the carboxylate group-containing aromatic amine as the component (C) which is a polymerization initiator component.

The monomer (A) is represented by the above formula (1). In the formula (1), R¹ is H or CH₃, and R² and R³ are each an inert group essentially composed of C and H. The term "inert group" means an organic group which can be stably kept without causing a bonding reaction or a cleavage reaction during the polymerization reaction or adhesion bonding reaction of the adhesive composition (other functional group bonded to the inert group may react). Examples of R² and R³ which are not particularly limited in that sense include ethylene and polyethylene (-(CH₂)ₙ-) groups (n is preferably an integer of 1 to 10), vinylene (-CH=CH-) group, divalent cyclo groups such as cyclohexene, divalent aromatic ring groups such as phenylene group, combinations thereof and groups containing a substituent as a branch or a relatively inert hetero atom such as halogen group or ether oxygen. A nitrogen atom is not preferred but stable atoms such as peptide bond are acceptable. Preferably, R² is - (CH₂)ₙ (n is an integer of 1 to 10) and R³ is -CH₂-, -CH=CH-, -C₆H₁₀- or -C₆H₄-.

When the molecular weight of the monomer (A) becomes too high, its dispersion force into the dentine may lower disadvantageously. Therefore, the molecular weight is preferably 400 or less, more preferably 300 or less.

Specific examples of the monomer (A) include (meth)acryloyloxypolymethylene(n = 2 to 10)succinic acids such as 2-(meth)acryloyloxyethylsuccinic acid ((meth)acryloyloxy in this text means acryloyloxy or methacryloyloxy), 3-(meth)acryloyloxypropylsuccinic acid and 4-(meth)acryloyloxybutylsuccinic acid; (meth) acryloyloxypolymethylene (n = 2 to 10) maleic acids such as 2-(meth)acryloyloxyethylmaleic acid, 3-(meth)acryloyloxypropylmaleic acid and 4-(meth)acryloyloxybutylmaleic acid; (meth)acryloyloxypolymethylene(n = 2 to 10)hexahydrophthalic acids such as 2-(meth)acryloyloxyethylhexahydrophthalic acid, 3-(meth)acryloyloxypropylhexahydrophthalic acid and 4-(meth)acryloyloxybutylhexahydrophthalic acid; and (meth)acryloyloxypolymethylene(n = 2 to 10)phthalic acids such as 2-(meth)acryloyloxyethylphthalic acid, 3-(meth)acryloyloxypropylphthalic acid and 4-(meth)acryloyloxybutylphthalic acid.

Out of these, 2- (meth) acryloyloxyethylsuccinic acid, 2-(meth)acryloyloxyethylmaleic acid, 2-(meth)acryloyloxyethylhexahydrophthalic acid and 2-(meth)acryloyloxyethylphthalic acid are particularly preferred.

The content of the monomer (A) is preferably 10 to 40 parts by weight, more preferably 15 to 30 parts by weight based on 100 parts by weight of the total of the components (A), (B), (C), (D) and (E).

The monofunctional radically polymerizable monomer (B) having a molecular weight of 220 or less and a boiling point of 60°C/10 mmHg or more contributes to the adhesion to the dentine of a resin cement and the stable storage of the resin cement. When the molecular weight is higher than 220, the dispersibility into the dentine of the monomer tends to lower. When the boiling point is lower than 60°C/10 mmHg, the monomer gradually transpires during storage, causing a change in the composition of the cement or a reduction in adhesion. Preferably, the molecular weight is 200 or less and the boiling point is 70°C/10 mmHg or more.

The monomer (B) is preferably a (meth)acrylate-based monomer. Examples of the methacrylate-based monomer include 2-hydroxyethyl methacrylate, 2-hydroxypropyl methacrylate, 3-hydroxypropylmethacrylate, 2-hydroxybutyl methacrylate, dimethylaminoethyl methacrylate, diethylaminoethyl methacrylate, glycidyl methacrylate, glycerin monomethacrylate, tetrahydrofurfuryl methacrylate, methoxyethylene glycol methacrylate, methoxydiethylene glycol methacrylate, methoxypropylene glycol methacrylate, butoxyethylene glycol methacrylate, hexyl methacrylate, 2-ethylhexyl methacrylate, cyclohexyl methacrylate, benzyl methacrylate, phenoxyethyl methacrylate and isobornyl methacrylate. Examples of the acrylate-based monomer include similar compounds as the above methacrylate-based monomers.

Out of these, monomers having a polar group such as 2-hydroxyethyl methacrylate, glycerin monomethacrylate and tetrahydrofurfuryl methacrylate are particularly preferred.

The content of the monomer (B) is preferably 2 to 30 parts by weight, more preferably 10 to 25 parts by weight based on 100 parts by weight of the total of the components (A), (B), (C), (D) and (E).

As for the carboxylic ester group-containing aromatic amine (C), the alkyl moiety of the ester is preferably a linear or branched alkyl having 2 to 10 carbon atoms, the aromatic ring is preferably a benzene ring, and the amine is preferably tertiary. The organic group of the amine is preferably a linear or branched alkyl having 1 to 4 carbon atoms, and further the carboxylic acid residue and the amine are preferably at the para-positions. The above linear or branched alkyl may be interrupted by an ether bond. Examples of the carboxylate group-containing aromatic amine (C) include alkyl p-dimethylaminobenzoates such as methyl p-dimethylaminobenzoate, ethyl p-dimethylaminobenzoate, butyl p-dimethylaminobenzoate, butoxyethyl p-dimethylaminobenzoate and isoamyl p-dimethylaminobenzoate; and alkyl p-diethylaminobenzoates such as methyl p-diethylaminobenzoate and ethyl p-diethylaminobenzoate.

Out of these, ethyl p-dimethylaminobenzoate and butoxyethyl p-dimethylaminobenzoate are particularly preferred.

The content of the carboxylic ester group-containing aromatic amine (C) is preferably 0.3 to 3 parts by weight, more preferably 0.5 to 2 parts by weight based on 100 parts by weight of the total of the components (A), (B), (C), (D) and (E).

Preferably, the photopolymerization initiator (D) is, for example, what can initiate the polymerization of a radically polymerizable monomer upon exposure to visible Irradiation. Examples of the photopolymerization initiator (D) include optical sensitizers such as benzoins including benzoin, benzoin methyl ether, benzoin ethyl ether and benzoin isopropyl ether; α-diketones including benzyl, 4,4'-dichlorobenzyl, diacetyl, α-cyclohexanedione, d,l-camphorquinone (CQ), camphorquinone-10-sulfonic acid and camphorquinone-10-carbxoylic acid; diphenyl monoketones including benzophenone, methyl benzoylbenzoate and hydroxybenzophenone; thioxanthones including 2,4-diethylthioxanthone and 2-isopropylthioxanthone; and acylphosphine oxides including 2,4,6-trimethylbenzoyldiphenylphosphine oxide. These photopolymerization initiators may be used alone or in combination. Out of these, α-diketones such as benzyl, 4,4'-dichlorobenzyl, diacetyl, α-cyclohexanedione, d,1-camphorquinone (CQ), camphorquinone-10-sulfonic acid and camphorquinone-10-carboxylic acid, and acylphosphine oxides are preferred. d,l-camphorquinone (CQ), camphorquinone-10-carboxylic acid and 2,4,6-trimethylbenzoyldiphenylphosphine oxide are particularly preferred.

The content of the photopolymerization initiator (D) is preferably 0.1 to 1 part by weight, more preferably 0.2 to 0.8 part by weight based on 100 parts by weight of the total of the components (A), (B), (C), (D) and (E).

Examples of the bifunctional radically polymerizable monomer (E) include poly(meth)acrylates of an alkanepolyol such as ethylene glycol di(meth)acrylate, propylene glycol di(meth)acrylate, butylene glycol di(meth)acrylate, neopentyl glycol di(meth)acrylate, hexylene glycol di(meth)acrylate, 2-hydroxypropyl di(meth)acrylate, trimethylolpropane tri(meth)acrylate and pentaerythritol tetra(meth)acrylate; polyoxyalkane polyol poly(meth)acrylates such as diethylene glycol di(meth)acrylate, triethylene glycol di(meth)acrylate, polyethylene glycol di(meth)acrylate, dipropylene glycol di(meth)acrylate, polypropylene di(meth)acrylate, dibutylene glycol di(meth)acrylate and dipentaerythritol hexa(meth)acrylate; di(meth)acrylates represented by the following formula (2): 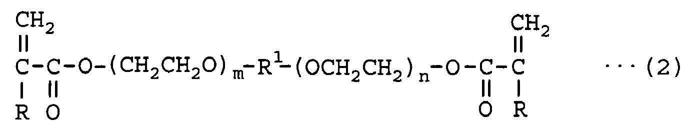 (wherein R is a hydrogen atom or methyl group, m and n are the same or different and each an integer of 0 to 10, and R¹ is an aliphatic or aromatic group represented by 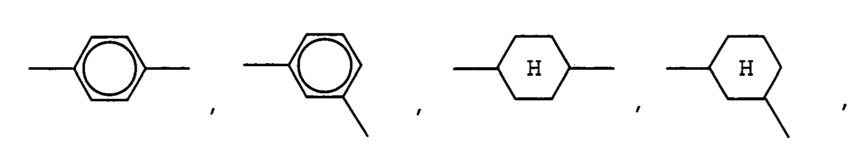 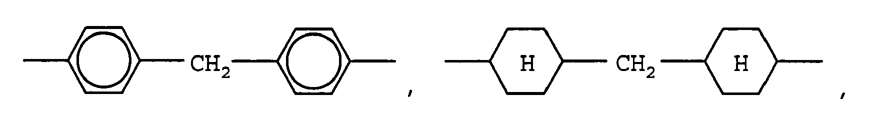 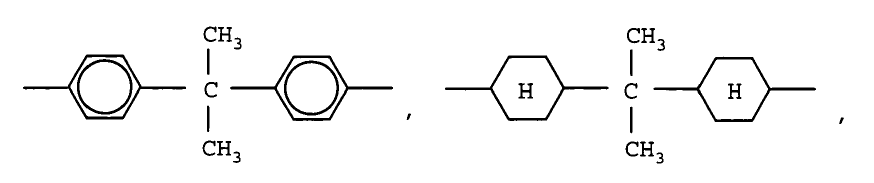 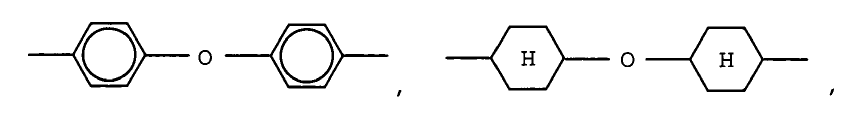 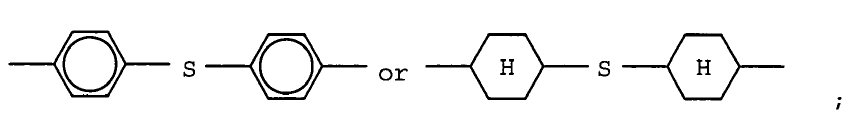 epoxy di (meth) acrylates represented by the following formula (3) : 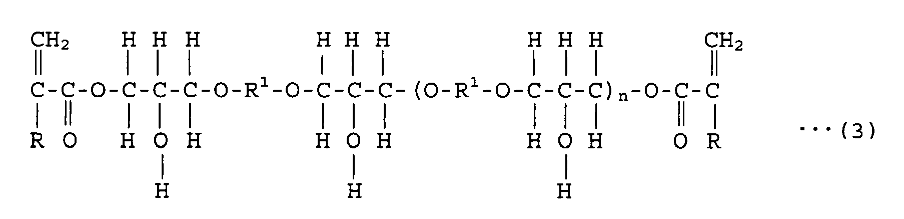 (wherein R is a hydrogen atom or methyl group, n is an integer of 0 to 10, and R¹ is an aliphatic or aromatic group represented by 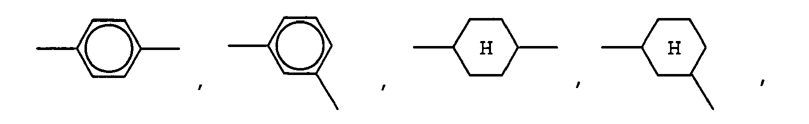 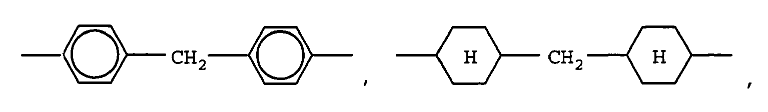 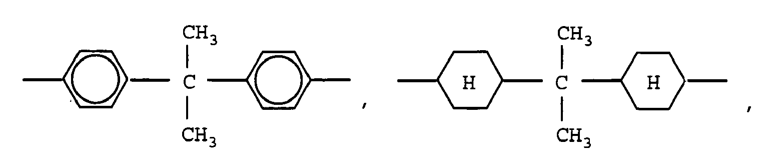 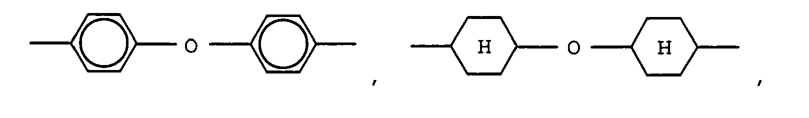 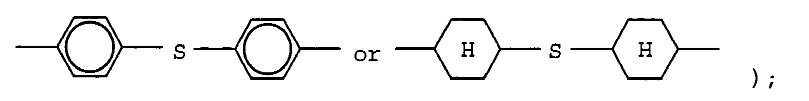 and polyfunctional (meth)acrylates having an urethane bond in the molecule represented by the following formula (4): 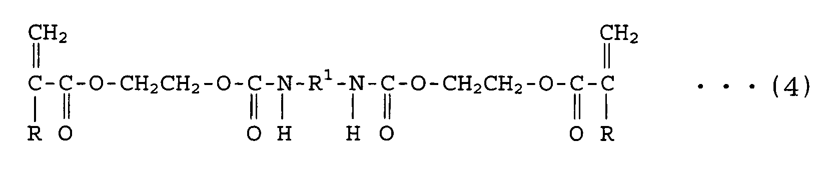 (wherein R is a hydrogen atom or methyl group, and R¹ is represented by

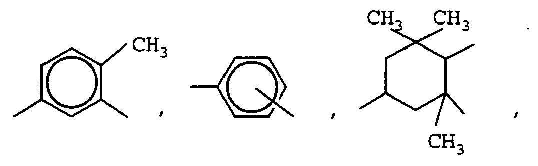

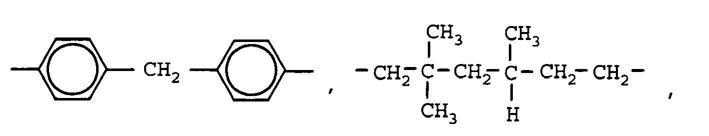

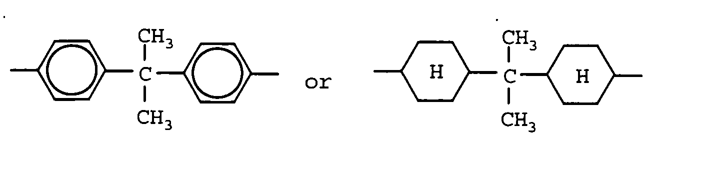

The bifunctional (meth)acrylate is particularly preferably a di(meth)acrylate having an ethylene glycol chain in the molecule such as triethylene glycol di(meth)acrylate or polyethylene glycol di(meth)acrylate, 2-hydroxypropyl di (meth) acrylate, a compound represented by the following formula (2)-a: 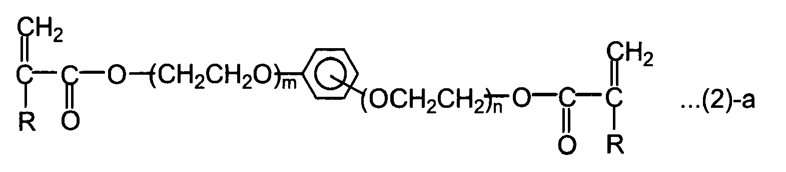 (wherein R, m and n are as defined in the above formula (2)), a compound represented by the following formula (3)-a: 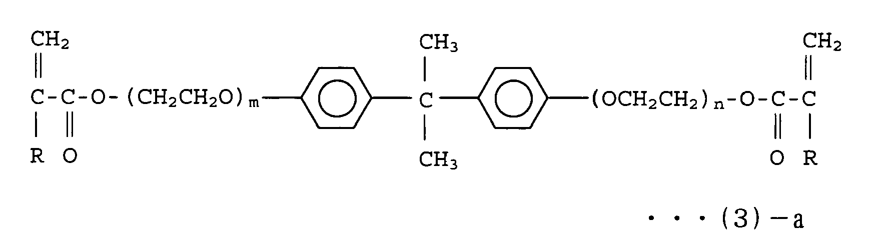 (wherein R, m and n are as defined in the above formula (2)), or a compound represented by the following formula (4)-a: 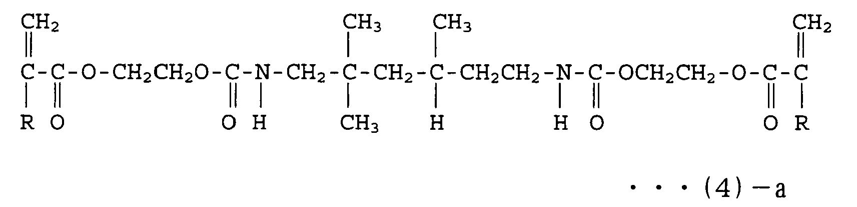 (wherein R is as defined in the above formula (4)). They may be used alone or in combination of two or more.

The content of the bifunctional monomer is preferably 50 to 80 parts by weight, more preferably 55 to 75 parts by weight based on 100 parts by weight of the total of the components (A), (B), (C), (D) and (E).

The filler (F) optionally used in the present invention may be an organic filler, inorganic filler, or a mixture or composite thereof. Examples of the inorganic filler (F1) include metal oxide powders such as zirconium oxide, bismuth oxide, titanium oxide, zinc oxide and aluminum oxide particles, metal chloride powders such as calcium carbonate, bismuth carbonate, calcium phosphate, zirconium phosphate, barium sulfate, sodium fluoride and calcium fluoride, glass fillers such as silica fine particle-, silica-, barium- and aluminum-containing glass powders, strontium-containing glass powders and zirconium silicate powder particles, and the above fillers having silver gradually releasing properties or fluorine gradually releasing properties. These inorganic fillers may be used alone or in combination.

To obtain firm connection between an inorganic filler and a resin matrix, an inorganic filler subjected to a surface treatment such as silane treatment is preferably used.

Examples of the organic filler (F2) include non-crosslinkable polymers such as polymethyl (meth)acrylate, polyethyl (meth)acrylate, methyl (meth)acrylate·ethyl (meth)acrylate copolymer, methyl (meth)acrylate·butyl (meth)acrylate copolymer and methyl (meth)acrylate·styrene copolymer, and (meth)acrylate polymers such as methyl (meth)acrylate·ethylene glycol di(meth)acrylate copolymer, methyl (meth)acrylate·triethylene glycol di(meth)acrylate copolymer and a copolymer of methyl (meth)acrylate and a butadiene-based monomer.

These fillers may be used alone or in combination. To obtain the suitable viscosity and operation ease of the cement composition, the average particle diameter of the above filler particles is preferably in the range of 0.005 to 50 µm, more preferably 0.01 to 30 µm.

The content of the filler (F) in the composition of the present invention is preferably 150 to 400 parts by weight, more preferably 200 to 300 parts by weight based on 100 parts by weight of the total of the components (A), (B), (C), (D) and (E).

The composition of the present invention may further contain a solvent, pigment, viscosity controller and polymerization inhibitor in limits not prejudicial to the object of the present invention.

The composition of the present invention is suitable for a dental manicure or a kit thereof. More specifically, it can be advantageously used in a dental manicure kit which may contain a dental coating material used to improve or protect the aesthetic properties of a tooth, for example, giving luster to a tooth or controlling a color by applying it to a tooth, a dental etching agent, a dental primer and a dental adhesive.

When the composition of the present invention is used for the above purpose, there can be provided a dental manicure kit which can determine the color of a tooth safely in a short period of time in advance and can change the color of a tooth without chipping off the surface of the dentine. There can be further provided a dental manicure kit which can improve the luster of a tooth by treating it therewith.

A specific example of the former dental manicure kit is a dental manicure kit which forms a hard film on the surface of the enamel of a tooth and contains a dental coating composition (p) contained in a container independently and containing a polymerization initiator capable of forming a cured film.

Preferably, the above dental coating composition (p) further contains (F) a filler and/or (G) a colorant.

The above dental coating composition (p) contains a top coating layer forming material (p-1) for forming the outermost layer and an opaque coating layer forming material (p-2) for forming a layer underlying the top coating layer. The top coating layer forming material and the opaque coating layer forming material are preferably charged into independent containers.

The Vickers hardness of a hard film formed from the above dental coating composition (p) is preferably in the range of 10 to 60 HV.

Preferably, the above dental manicure kit contains at least one component selected from the group consisting of (q) a dental etching agent, (r) a dental primer and (s) a dental adhesive independently of the above dental coating composition (p).

The total thickness of a layer or a film formed on the surface of the enamel of the above tooth is preferably in the range of 10 to 400 µm.

After the surface of a tooth is treated with a dental etching agent (q) of the above dental manicure kit, the following treatment is preferably carried out.

In the above manicure kit, the dental coating composition (p) contains at least one of the top coating layer forming material (p-1) for forming the outermost layer, the opaque coating layer forming material (p-2) for forming a layer underlying the top coating layer and the dental adhesive (s).

The composition of the present invention may consist of the top coating layer forming material (p-1) for forming the outermost layer. In general, this top coating layer forming material (p-1) becomes a transparent hard film. By mixing together a polymerizable monomer (a), a polymerization initiator (b), a filler (F) and a colorant (G), the opaque coating layer forming material (p-2) may be prepared. This opaque coating layer forming material (p-2) forms a layer underlying the above top coating later forming material (p-1).

The top coating layer forming material (p-1) and the opaque coating layer forming material (p-2) are charged into separate containers.

The above dental coating composition (p) may contain a colorant (G). The colorant (G) may be a known dental pigment or dye.

The pigment may be an inorganic pigment such as black iron oxide, yellow iron oxide, red iron oxide, titanium yellow or titanium white, or an organic pigment such as bromophthal red and bromophthal yellow. The content of the colorant is preferably 0.01 to 3 parts by weight based on 100 parts by weight of the total of the dental coating composition (p) and the filler (F).

The dental coating composition (p) contained in the first container of the above dental manicure kit may be mixed with other additives in limits not prejudicial to the object of the present invention. The additives include a bactericide, a stabilizer and a polymerization inhibitor.

In the dental manicure kit, the above dental coating composition (p) may be divided into an opaque coating layer forming material (p-2) containing a colorant and a transparent coating layer forming material (p-1) containing no colorant both of which are contained in separate containers.

As means of controlling bonding strength to the enamel, the existence of the etching of the surface of a tooth, the type of an etching agent, an etching method and the application of a primer or an adhesive before the application of the dental coating composition (p) may be selected.

Before the use of the dental manicure kit of the present invention, the tooth is preferably subjected to a pre-treatment.

The pre-treatment which is carried out before the use of the dental manicure kit is, for example, the etching of the mating surface with (q) a dental etching agent, the modification of the mating surface with (r) a dental primer, or the etching and modification of the mating surface with a primer having etching ability.

The dental etching agent (q) used for the etching of the surface of a tooth is, for example, an aqueous solution containing 5 to 60 wt% of phosphoric acid or an aqueous solution containing 10 wt% of citric acid and 3 wt% of ferric chloride.

This dental etching agent (q) is charged into a container separate from that of the above dental coating agent (p) to constitute the dental manicure kit of the present invention.

When the dental manicure kit of the present invention is used, the surface of a tooth which has been etched with the above dental etching agent (q) is preferably treated with a separately prepared dental primer. This dental primer is an agent for modifying the mating surface of a tooth, and the dental manicure kit of the present invention may include a container containing the dental primer (r) which is an agent for modifying the mating surface of a tooth.

Out of the dental primers (r) used in the present invention, an aqueous solution containing 20 to 50 wt% of 2-hydroxyethyl (methyl)acrylate and 1,3-dihydroxypropyl mono (meth) acrylate is used as the dental primer (r) for the modification of the mating surface.

In the present invention, a primer having etching ability used for etching and modifying the mating surface may also be used as the dental primer. The primer having etching ability is preferably an aqueous solution containing an organic acid (including a monomer having an acid group) and a component for modifying the delimed dentine and promoting the dispersion of an adhesive into the dentine. Examples of the component for promoting the dispersion of an adhesive into the dentine include hydroxyl group-containing monomers such as alkylene glycol, polyalkylene glycol, 2-hydroxyethyl (meth)acrylate and 1,3-dihydroxypropyl mono(meth)acrylate and polyethylene glycol (meth)acrylate.

The dental manicure kit of the present invention may include a container containing a separately prepared adhesive layer forming component (s).

The above adhesive layer forming component (s) may contain other additives in limits not prejudicial to the object of the present invention. The additives include a solvent such as water, acetone or ethanol, a thickener, a bactericide and a stabilizer.

The above dental manicure kit consists of a container containing the above dental coating layer forming material (p) as an essential container, a container containing an etching agent (q), a container containing a primer (r) and a container containing an adhesive (s), and the opaque coating layer forming material (E) is prepared separately or included in the kit. The above dental coating layer forming material (p) can be divided into a transparent coating layer forming component (p-1) containing no colorant and no filler and an opaque coating layer forming material (p-2) containing a colorant and a filler and forming a layer underlying the transparent coating layer forming component (p-1). When the dental coating layer forming material (p) is divided into the transparent coating layer forming component (p-1) and the opaque coating layer forming material (p-2), the transparent coating layer is preferably made harder than the opaque coating layer.

The opaque coating layer forming material (p-2) can be applied repeatedly for color matching. The aesthetic properties of the hard film may be impaired in a relatively short period of time. In this case, the transparent coating layer forming component (p-1) may be applied to the hard film to repair it.

As for the thickness of each layer of the dental manicure, when the opaque coating layer containing a colorant is to be formed, the opaque coating layer is preferably as thick as 20 to 300 µm from the viewpoints of its covering power and operation efficiency. When a hard film for improving abrasion resistance is formed, the thickness of a transparent top coating layer is preferably 5 to 100 µm.

When an adhesive is used to improve adhesion durability in the mouth, the thickness of the adhesive layer is preferably 3 to 100 µm. The thickness of the primer layer is preferably 3 µm or less.

The thickness of the entire manicure laminate structure formed from the dental manicure kit of the present invention is preferably 10 to 400 µm, more preferably 20 to 250 µm, most preferably 30 to 100 µm from the viewpoints of giving no feeling that something is wrong, meeting between opposed teeth and easy removal by a scaler.

The surface hardness (Vickers hardness) of the coating layer formed from the dental manicure kit of the present invention is preferably 10 to 60 HV, more preferably 20 to 60 HV. When the surface hardness is lower than 10 HV, the surface luster is gone quickly by cleaning with a toothbrush due to low abrasion resistance, thereby causing an aesthetic problem.

When the dental manicure kit of the present invention is used, the dental coating composition may be applied to the enamel directly or after the treatment of the surface of a tooth. Multi-coating technique may be carried out to adjust the color of the surface of a tooth. When an adhesive is used to improve the durability in the mouth, the adhesive layer is formed and then the coating layer is formed on the adhesive layer. When an etching agent is used to further improve the durability, the etching agent is applied before the use of the adhesive. When the opaque coating layer and the transparent coating layer are formed separately to improve the abrasion resistance, the transparent coating layer is formed on the opaque coating layer.

The above dental coating composition, transparent coating layer forming component, opaque coating layer forming component, adhesive layer forming component and primer are generally cured by a photopolymerization reaction. For this curing, a dental halogen light illuminator is used to apply visible light. When a curing reaction is carried out by using visible light, the illumination time of visible light is generally 10 to 60 seconds, preferably 15 to 40 seconds.

The containers for containing the components of the dental manicure kit of the present invention must contain each component independently to prevent mixing with another component.

The above dental manicure kit of the present invention can improve the color of the surface of the dentine safely in a short period of time without chipping off the surface of the dentine. The coating layer thus formed can be easily removed as required because it does not have high bonding strength to the dentine.

### Examples

The following examples are provided for the purpose of further illustrating the present invention but are in no way to be taken as limiting.

### (Conditions for carrying out the invention)

### (A. storage stability of adhesive composition)

The adhesive composition was put into a syringe which was kept in a 76°C thermostat box for 24 hours to be heated, and it was checked how the composition was squeezed out from the syringe. When it could be squeezed out in the same manner as before the heat treatment and no change in the properties of the adhesive composition was seen, the storage stability of the adhesive composition was evaluated as ○. When it could be squeezed out from the syringe but a change in the properties of the adhesive composition was observed such that it had a hard core, or when it could not be squeezed out from the syringe, the storage stability was evaluated as ×.

### (B. measurement of bonding strength to enamel)

(1) The front tooth of a bovine lower forehead was polished with 1000# abrasive paper under running water to chip off a flat enamel surface.
(2) The obtained enamel mating surface was treated with the Red enamel surface treating material (of Sun Medical Co., Ltd.), rinsed with water and dried to specify a mating area with a 3.0 mm-diameter tape.
(3) The mating surface was made uneven (lattice-like grooves having a width of 0.25 mm, a depth of 0.17 mm and a pitch of 0.5 mm, end portions of the mating surface were chamfered (width of 0.125 mm, depth of 0.17 mm)), and the adhesive composition was placed on a SUS304 metal rod (diameter of 4 mm, length of 15 mm) which was sandblasted, brought into pressure contact with the surface of the tooth specified with the tape and illuminated by an illuminator for 20 seconds to be cured and bonded.
(4) 20 hours after the above bonded sample was put into a thermostat tank having a temperature of 37°C and a humidity of 100 %, its tensile bonding strength was measured, or the sample was put into a thermal cycle tester to be heated at 5°C and 55°C to measure its durability bonding strength under tension after 5,000 thermal cycles.

### (C. constituent components of adhesive composition)

The constituent components of the adhesive composition are shown by the following abbreviations.
- MESA:: 2-methacryloyloxyethylsuccinic acid
- MEHA:: 2-methacryloyloxyethylhexahydrophthalic acid
- MEFA:: 2-methacryloyloxyethylphthalic acid
- 4-META:: 4-methacryloyloxyethyltrimellitic anhydride
- HEMA:: 2-hydroxyethyl methacrylate
- GCMA:: glycerin monomethacrylate
- TFMA:: tetrahydrofurfuryl methacrylate
- EMA:: ethyl methacrylate
- DMABE:: ethyl p-dimethylaminobenzoate
- DMABEB:: butoxyethyl p-dimethylaminobenzoate
- DMPT:: p-dimethylaminotoluene
- CQ:: camphorquinone
- UDMA:: di(methacryloxyethyl)trimethylhexamethylene diurethane
- RDMA:: resorcin dimethacrylate
- NaF:: sodium fluoride
- FSi:: silica fine particles (average particle diameter of 14 nm)
- GF1:: glass filler (SiO₂: 30, SrO:20, F:15, ZnO:10, P₂O₅<5, Na₂O<5 (numerical values show wt%), average particle diameter of 3.5 µm)
- GF2:: glass filler (SiO₂: 50, BaO:25, B₂O₃:10, Al₂O₃:10, F<2 (numerical values show wt%), average particle diameter of 14 µm)

### Examples 1 to 6

When the storage stabilities and bonding strengths to the enamel of adhesive compositions shown in Table 1 were measured, they were all satisfactory.

### Comparative Examples 1 and 2

When the storage stabilities and adhesions of adhesive compositions obtained by changing the content of the component (A) of the composition of Example 1 as shown in Table 1 were measured, no bad influence upon storage stability was seen but a great reduction in adhesion was observed.

### Comparative Examples 3 and 4

When the storage stabilities and adhesions of compositions obtained by changing only the content of the component (B) of the composition of Example 1 as shown in Table 1 were measured, no bad influence upon storage stability was seen but a great reduction in adhesion was observed.

### Comparative Example 5

When the storage stability and adhesion of a composition obtained by changing only the component (A) of the composition of Example 1 as shown in Table 1 were measured, a great reduction in storage stability was seen.

### Comparative Example 6

When the storage stability and adhesion of a composition obtained by changing only the component (B) of the composition of Example 1 as shown in Table 1 were measured, a great reduction in storage stability was seen.

### Comparative Example 7

When the storage stability and adhesion of a composition obtained by changing only of the component (C) of the composition of Example 1 as shown in Table 1 were measured, a great reduction in storage stability was seen.

**Table 1**

| | Cement composition (:parts by weight) | | | | | | Storage stability | Bonding strength (MPa) | |
|---|---|---|---|---|---|---|---|---|---|
| | Component (A) | Component (B) | Component (C) | Component (D) | Component (E) | Component (F) | | Right after | Durability bonding strength |
| Ex.1 | MEFA:8 | HEMA:5 | DMABEB:0.4 | CQ:0.2 | UDMA:14 RDMA:10 | FSi:4,NaF:0.5 GF1:30,GF2:65 | ○ | 12 | 10 |
| Ex.2 | MESA:8 | HEMA:9 | DMABEB:0.4 | CQ:0.2 | UDMA:14 RDMA:10 | FSi:5, NaF:0.5 GF1:20, GF2:80 | ○ | 13 | 9 |
| Ex.3 | MEHA:8 | HEMA:9 | DMABEB:0.4 | CQ:0.2 | UDMA:14 RDMA:10 | FSi:5, NaF:0.5 GF1:20, GF2:80 | ○ | 14 | 10 |
| Ex.4 | MEFA:8 | HEMA:9 | DMABEB:0.4 | CQ:0.2 | UDMA:14 RDMA:10 | FSi:5, NaF:0.5 GF1:20, GF2:80 | ○ | 13 | 9 |
| Ex.5 | MEFA:8 | GCMA:9 | DMABEB:0.4 | CQ:0.2 | UDMA:14 RDMA:10 | FSi:5, NaF:0.5 GF1:20, GF2:80 | ○ | 11 | 9 |
| Ex.6 | MEFA:8 | TFMA:7 | DMABEB:0.4 | CQ:0.2 | UDMA:14 RDMA:10 | FSi:5, NaF:0.5 GF1:20, GF2:80 | ○ | 10 | 9 |
| C.Ex.3 | MEFA:8 | HEMA:0 | DMABEB:0.4 | CQ:0.2 | UDMA:14 RDMA:10 | FSi:4, NaF:0.5 GF1:30, GF2:65 | ○ | 6 | 3 |
| C.Ex.4 | MEFA:8 | HEMA:20 | DMABEB:0.4 | CQ:0.2 | UDMA:14 RDMA:10 | FSi:4, NaF:0.5 GF1:30, GF2:65 | ○ | 15 | 5 |
| C.Ex.5 | 4-META:8 | HEMA:5 | DMABEB:0.4 | CQ:0.2 | UDMA:14 RDMA:10 | FSi:4, NaF:0.5 GF1:30, GF2:65 | × | - | - |
| C.Ex.6 | MEFA:8 | EMA:5 | DMABEB:0.4 | CQ:0.2 | UDMA:14 RDMA:10 | FSi:4, NaF:0.5 GF1:30, GF2:65 | × | - | - |
| C.Ex.7 | MEFA:8 | HEMA:5 | DMPT:0.4 | CQ:0.2 | UDMA:14 RDMA:10 | FSi:4, NaF:0.5 GF1:30, GF2:65 | × | - | - |

| | | | | | | | | | |
|---|---|---|---|---|---|---|---|---|---|
| (a trace amount of a hydroquinone-based stabilizer was blended in each Example and Comparative Example) | | | | | | | | | |

### Example 7

The adhesive composition of Example 1 was used as the opaque coating layer forming material (p-2), and a dental manicure coating was formed by applying a dental adhesive (s) containing no component (F) of the adhesive composition of Example 1, the dental adhesive (s) as the top coating layer forming material (p-1), the opaque coating layer forming material (p-2) and the top coating layer forming material (p-1) in the mentioned order.

The durability bonding strength was 10 MPa and no irregular color was observed.

By using the photocurable dental enamel adhesive composition of the present invention, excellent adhesion and durability can be obtained without a treatment with a priming material or an adhesive aid for bonding to the enamel as described above.

## Claims

1. A photocurable dental enamel adhesive composition comprising (A) a radically polymerizable monomer having an acid group and a structure represented by the following formula (1):
CH₂=CR¹-COO-R²-OCO-R³-COOH (1)
(R¹ is H or CH₃, and R² and R³ are each independently a divalent inert group essentially composed of C and H), (B) a monofunctional radically polymerizable monomer having a molecular weight of 220 or less and a boiling point of 60°C/10 mmHg or more, (C) a carboxylic ester group-containing aromatic amine, (D) a photopolymerization initiator and (E) a bifunctional radically polymerizable monomer.

2. The enamel adhesive composition according to claim 1 which further comprises (F) a filler.

3. The enamel adhesive composition according to claim 1 which comprises 10 to 40 parts by weight of the component (A), 2 to 30 parts by weight of the component (B), 0.3 to 3 parts by weight of the component (C) , 0.1 to 1 part by weight of the component (D) and 50 to 80 parts by weight of the component (E) based on 100 parts by weight of the total of the components (A), (B), (C), (D) and (E).

4. The enamel adhesive composition according to claim 2 which comprises 150 to 400 parts by weight of the component (F) based on 100 parts by weight of the total of the components (A), (B), (C), (D) and (E).

5. The enamel adhesive composition according to any one of claims 1 to 4, wherein the component (A) is
2-methacryloyloxyethylsuccinic acid and/or
2-methacryloyloxyethylphthalic acid.

6. The enamel adhesive composition according to any one of claims 1 to 5, wherein the component (B) is 2-hydroxyethyl methacrylate and/or 2-hydroxypropyl methacrylate.

7. The enamel adhesive composition according to any one of claims 1 to 6, wherein the component (C) is ethyl p-dimethylaminobenzoate and/or butoxyethyl p-dimethylaminobenzoate.
